# EUROPEAN PATENT SPECIFICATION

(11) **EP 0 732 084 B1**
(45) Date of publication and mention of the grant of the patent: **07.07.2004**
(21) Application number: 96200666.4
(22) Date of filing: 12.03.1996
(51) Int. Cl.: A61F 2/06, A61B 18/00

(54) **Assembly comprising a balloon catheter with a single lumen catheter and a light conductor**
Anordnung entaltend einen einlumigen Ballonkatheter und einen Lichtleiter
Ensemble comprenant un cathéter à ballon à lumière simple et un conduit de lumière

(30) Priority: 13.03.1995 NL 9500495
(43) Date of publication of application: 18.09.1996
(73) Proprietor: CORDIS EUROPA N.V., NL-9301 LJ Roden (NL)
(72) Inventor: Van Werven-Franssen, Gerda Hendrika Maria, 9301 EK Roden (NL); Ligtenberg, Hendrikus Cornelis Geert, 9351 PX Leek (NL); Haan, Marcel Gerhard, 9312 PE Nietap (NL); Leone, James Ernest, 33156 Miami, Florida (US)
(74) Representative: 't Jong, Bastiaan Jacob

(56) References cited:
- EP-A- 0 311 458
- EP-A- 0 367 516
- EP-A- 0 411 132
- EP-A- 0 611 582
- EP-A- 0 646 360
- WO-A-83/03188
- WO-A-94/24962
- US-A- 4 773 899
- US-A- 5 100 429
- US-A- 5 125 925

## Description

Document US-A-5 100 429 discloses a catheter comprising a body with two lumens, a balloon of a tight-transmitting material, a flexible light conductor, and a stent of a plastic material that cures due to the action of light, the balloon having a proximal section arranged on the distal end of the body and a distal section provided with a separate section to which the distal end of the light conductor is fixed.

The invention is defined in claim 1. Preferred embodiments are defined in the dependent claims.

The catheter can be inserted into the patient and its balloon can be placed in the required position by introducing a stiff guide wire through the lumen, until the end of the guide wire is placed against the distal end of the balloon member, and stretches the latter in line with the basic body. After placing the balloon in the right position, the guide wire is removed and a light conductor, in particular an optic fibre bundle, is introduced via the lumen of the basic body. The end of this optic fibre bundle has been treated in such a manner that light, which is admitted into the proximal end of the fibre bundle, is emitted in a radial direction at the distal end situated inside the balloon. This can be achieved by providing the distal end of the light conductor with a somewhat roughened surface, for instance by means of grinding. This end-section can be made of solid light-conducting material, whereas the section extending from that end-section to the proximal end-section is made of an optic fibre bundle.

With the radially emitted light a stent, made of a plastic material which cures under the action of light, and which is expanded by the balloon, can be cured so that it retains its ultimate expanded shape. A suitable material for the plastic stent is a material which cures under the action of UV-light. UV-light is supplied, via the light conductor, from the proximal end to the site of the stent.

A preferred embodiment is characterised in claim 2. On introducing the catheter, it is the light conductor which straightens the balloon member and keeps it in line with the basic body. At the same time this embodiment ensures that the distal end of the light conductor is placed in the right position inside the balloon member, so that one can be certain that the light is supplied to the right place.

In order to be able to advance the balloon to the correct position inside the body of the patient, the features as set out in claim 3 are preferably employed.

A suitable embodiment is characterised in claim 4. If the stent itself is also visible under NMR-conditions and/or when using X-radiation, for instance by incorporating a suitable filler in the plastic material of which the stent has been made, the relative position of the working end of the light conductor in relation to the stent can be looked at and checked carefully in a catheterization laboratory.

An advantageous embodiment is characterised in claim 5. In that case the catheter can be introduced by passing it over a guide wire.

The invention will be explained in greater detail in the following description with reference to the attached drawings.
- Figure 1: shows a partly broken away perspective view of an assembly according to the invention.
- Figure 2: shows the balloon member of the catheter of figure 1 when positioning a stent.
- Figure 3: shows the distal end-section of the assembly of Figure 1.
- Figure 4: shows yet again another embodiment of the assembly of Figure 1.

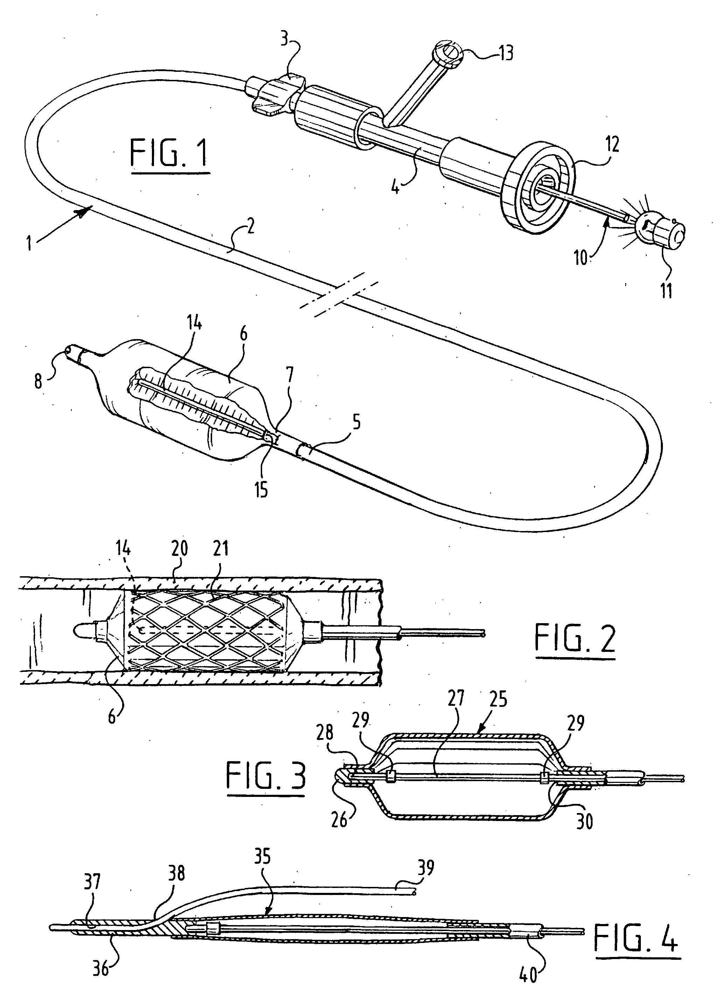

The assembly illustrated in figure 1 comprises a catheter 1 with a light conductor 10 received inside it.

The catheter 1 comprises a tube-like basic body 2 with one single lumen 15. At the proximal end, that is to say the end which remains outside the body of the patient during treatment, a connecting member has been arranged in the form of a Luerlock-connection, known as such. The connecting member 3 is connected with a haemostatic device 4, known as such, which will be explained in greater detail below.

At the distal end 5 of the basic body 2 a balloon member 6 has been arranged. The relatively proximal end-section 7 has been arranged on the distal end 5 of the basic body 2. The opposite, relatively distal end 8 of the balloon member 6 is closed. Consequently the inside of the balloon member 6 is free of the basic body 2.

The haemostatic device 4 comprises in the usual manner a central channel which is connected with the lumen of the catheter connected to it. Through the connection 12, an elongated element, in this case a light conductor 10, can be introduced in a sealed manner. Via the connection 13 liquid or gas under pressure, for the purpose of expanding the balloon member 6, can be supplied.

The light conductor 10 has been made in such a way that the light which is admitted into the proximal end by a source of light 11, schematically illustrated in figure 1, is emitted in a radial direction at the distal end 14. As a result it is possible to conduct light from the proximal end of the assembly to the balloon member 6.

The assembly of figure 1 is used for the purpose of fitting a stent, made of a plastic material curing under a certain type of light, inside a blood vessel. This has been schematically illustrated in figure 2.

The blood vessel 20 is for instance locally impaired, and this impairment is to be reinforced by means of a stent 21. The stent 21 used, has been made of a plastic material which cures due to the action of light, in particular UV-light. The stent 21 is introduced into the patient in compressed state, arranged around the balloon member 6. By means of marking rings, arranged on either side of the balloon member 6, the position of the balloon member and consequently that of the stent 21 can be visualized on for instance an X-ray screen. On introduction a stiff guide wire is for example used instead of the light conductor 10, which is positioned with its distal end against the relatively distal end 8 of the balloon member 6, so that the balloon member is stretched on introduction.

As soon as the stent is placed in the correct position, the guide wire is replaced by the light conductor 10 and liquid under pressure is supplied via the connection 13 in the haemostatic device, as a result of which the balloon 6 and consequently the stent 21 will expand. Next the source of light 11 is activated and the stent exposed to the light emitted radially from the end 14 of the light conductor 10. After a certain exposure time, the duration of which has been established experimentally as being sufficient to cure the stent, the light source 11 is switched off and the pressure inside the balloon 6 drained off; as a result the balloon will contract and the catheter can be removed completely, leaving the expanded stent in situ.

Figure 3 illustrates a portion of the aforementioned embodiment of the assembly according to the invention. The balloon 25 has been provided with a separate end-section 26 at its relatively distal end, in a depression of which the distal end 28 of a light conductor 27 has been fixed, for instance by glueing. The light conductor 27 thus forms a unit with the catheter and provides a certain bending stiffness at the balloon member 25, which enables the introduction of the catheter and in particular the balloon member thereof. The light conductor 27 is provided with two marking rings 29, which visualize the position of the balloon on for instance an X-ray screen. The light conductor 27 extends through the single lumen 30 of the basic body of the catheter and can be moved inside it.

With the embodiment of figure 4 the balloon member 35 is provided at its relatively distal end with a tube-like end- section 36, which extends over a certain distance in front of the balloon 35. This tube-like end-section 36 has been provided with a guiding lumen 37 which extends from the tip to an opening 38 in the wall of the end-section 36 close to the balloon 35. Through this channel 37 a guide wire 39 can be advanced, over which a catheter can be passed and introduced into a patient. Also with this embodiment the basic body 40 does not extend further than the relatively proximal end of the balloon member 35.

## Claims

1. An assembly of a catheter (1), a stent (21) and a light conductor (27), wherein
the catheter (1) comprises a tubular body (2) with a proximal and distal end (5) and provided with a single lumen (15) and a balloon member (6) made of a light-transmitting material, wherein
the stent is made of a plastic material that cures due to the action of light, and wherein
the light conductor (27) is flexible and rod-shaped and comprises light conducting material and has a smaller diameter than the lumen (15) of the body (2) and a length greater than the length of the catheter (1),
the balloon member having an end-section arranged on the distal end (5) of the body (2) and an opposite distal end-section (8) which is closed and is provided with a separate end section (26) with a depression in which the distal end (28) of the light conductor (27) is fixed.

2. An assembly as claimed in claim 1, wherein the light conductor (27) extends from the proximal end through the lumen (30) to the distal end (26) of the balloon member (6).

3. An assembly as claimed in claim 1 or 2, wherein close to the opposite end-sections of the balloon member (6) marking elements (29) are arranged which are visible under NMR-conditions and/or when using X-radiation.

4. An assembly as claimed in claim 3, wherein the marking elements (29) are arranged on the light conductor (27).

5. An assembly as claimed in claim 1, wherein the separate end section (36) comprises a guiding lumen (37) extending from a tip to an opening (38) in a wall of the separate end section (36) close to the balloon member (6).

## Patentansprüche

1. Anordnung eines Katheters (1), eines Stents (21) und eines Lichtleiters (27),
worin der Katheter (1) einen röhrenförmigen Körper (2) mit einem proximalen und einem distalen Ende (5) aufweist und versehen ist mit einem einzelnen Lumen (15) und einem Ballonteil (6), das aus lichtdurchlässigem Material hergestellt ist,
worin der Stent aus einem Kunststoffmaterial hergestellt ist, das aufgrund der Wirkung von Licht aushärtet, und
worin der Lichtleiter (27) flexibel und stabförmig ist und lichtleitendes Material aufweist und einen kleineren Durchmesser als das Lumen (15) des Körpers (2) und eine Länge größer als die Länge des Katheters (1) aufweist,
wobei das Ballonteil einen Endabschnitt, der auf dem distalen Ende (5) des Körpers (2) angeordnet ist, und einen entgegengesetzten Endabschnitt (8), der geschlossen ist und mit einem getrennten Endabschnitt (26) mit einer Vertiefung versehen ist, in der das distale Ende (28) des Lichtleiters (27) befestigt ist, aufweist.

2. Anordnung nach Anspruch 1, bei der sich der Lichtleiter (27) von dem proximalen Ende durch das Lumen (30) zu dem distalen Ende (26) des Ballonteiles (6) erstreckt.

3. Anordnung nach Anspruch 1 oder 2, bei der nahe zu den entgegengesetzten Endabschnitten des Ballonteils (6) Markierungselemente (29) angeordnet sind, die unter NMR-Bedingungen und/oder, wenn Röntgenstrahlen benutzt werden, sichtbar sind.

4. Anordnung nach Anspruch 3, bei der die Markierungselemente (29) auf dem Lichtleiter (27) angeordnet sind.

5. Anordnung nach Anspruch 1, bei der der getrennte Endabschnitt (36) ein Führungslumen (37) aufweist, das sich von einer Spitze zu einer Öffnung (38) in einer Wand des getrennten Endabschnittes (36) nahe zu dem Ballonteil (6) erstreckt.

## Revendications

1. Ensemble comprenant un cathéter (1), un stent (21) et un conducteur de lumière (27), dans lequel
le cathéter (1) comprend un corps tubulaire (2) avec une extrémité proximale et distale (5) et pourvu d'une lumière simple (15) et d'un élément formant ballonnet (6) fabriqué dans un matériau transmettant la lumière, dans lequel
le stent est fabriqué dans une matière plastique qui se durcit sous l'action de la lumière, et dans lequel
le conducteur de lumière (27) est flexible et en forme de tige et comprend un matériau photoconducteur et présente un diamètre inférieur à la lumière (15) du corps (2) et une longueur supérieure à la longueur du cathéter ( 1 ),
l'élément formant ballonnet présentant une section d'extrémité disposée sur l'extrémité distale (5) du corps (2) et une section d'extrémité distale opposée (8) qui est fermée et est pourvu d'une section d'extrémité séparée (26) avec un enfoncement dans lequel l'extrémité distale (28) du conducteur de lumière (27) est fixée.

2. Ensemble selon la revendication 1, dans lequel le conducteur de lumière (27) s'étend de l'extrémité proximale à travers la lumière (30) jusqu'à l'extrémité distale (26) de l'élément formant ballonnet (6).

3. Ensemble selon la revendication 1 ou 2, dans lequel à proximité des sections d'extrémité opposées de l'élément formant ballonnet (6) des éléments de marquage (29) sont disposés, lesquels sont visibles dans des conditions de RMN et/ou lors de l'utilisation du rayonnement X.

4. Ensemble selon la revendication 3, dans lequel les éléments de marquage (29) sont disposés sur le conducteur de lumière (27).

5. Ensemble selon la revendication 1, dans lequel la section d'extrémité séparée (36) comprend une lumière de guidage (37) s'étendant d'une pointe à une ouverture (38) dans une paroi de la section d'extrémité séparée (36) à proximité de l'élément formant ballonnet (6).
